# FASCICULE DE BREVET EUROPEEN

(11) **EP 0 448 418 B1**
(45) Date de publication et mention de la délivrance du brevet: **11.10.1995**
(21) Numéro de dépôt: 91400326.4
(22) Date de dépôt: 11.02.1991
(51) Int. Cl.: A61M 39/02, A61B 17/34, A61M 1/00

(54) **Perfectionnements aux dispositifs d'accès vasculaires implantables**
Verbesserungen an implantierbaren vaskularen Anschlussvorrichtungen
Improvements to implantable vascular access devices

(30) Priorité: 15.02.1990 FR 9001832
(43) Date de publication de la demande: 25.09.1991
(73) Titulaire: ANTHEOR, F-86130 Jaunay Clan (FR)
(72) Inventeur: ANTHEOR, F-86130 Jaunay Clan (FR)
(74) Mandataire: Lerner, François

(56) Documents cités:
- EP-A- 0 153 047
- DE-C- 3 618 390
- FR-A- 2 628 639
- FR-A- 2 645 029
- GB-A- 2 185 689
- US-A- 1 835 287
- US-A- 3 312 220
- US-A- 4 534 759
- US-A- 4 632 671
- US-A- 4 692 146

## Description

L'invention a pour objet des perfectionnements aux dispositifs d'accès vasculaires implantables.

Les accès vasculaires implantables sont utilisés principalement dans le cas où des opérations répétées de ponction (prélèvement), de dialyse (traitement), de traitement chimiothérapique, d'alimentation en perfusion ou d'autres opérations de ce type doivent être répétées périodiquement de nombreuses fois sur une longue période.

Les dispositifs connus comprennent essentiellement une chambre en forme de capsule plate logée sous la peau, communicant avec une veine, la chambre étant fermée du côté de la peau par une membrane dite "septum" en élastomère ou silicone qu'il est possible de perforer un grand nombre de fois sans altérer l'étanchéité du système. Pour ne pas détériorer le septum, la ponction doit être faite obligatoirement à l'aide d'aiguilles spéciales à tête en biseau, de petit diamètre, ne pouvant dépasser 0,7 mm de diamètre extérieur.

Un dispositif de ce type est par exemple décrit au brevet FR-A-2628639.

Cette limitation du diamètre des aiguilles utilisables constitue un inconvénient majeur ne permettant pas d'utiliser de tels accès vasculaires pour la perfusion de solutions à haute viscosité, notamment pour la nutrition ou divers traitements chimiothérapiques, ni pour la perfusion rapide de gros volumes, comme dans le cas de transfusions sanguines, ni pour le prélèvement de l'organisme du sang pour analyse ou épuration, en particulier dans un traitement de dialyse.

Dans ces conditions, pour toutes les interventions du type précité, et de nombreuses autres, on est obligé d'avoir recours à des cathéters extériorisés qui présentent de graves inconvénients, notamment au niveau du risque infectieux que leur emploi entraîne ou qui sont à l'origine d'accidents dus à des arrachements volontaires ou accidentels du cathéter, ces dispositifs étant de plus très inconfortables pour le patient.

L'invention a pour objet des perfectionnements aux dispositifs d'accès vasculaires implantables permettant l'utilisation de tels accès même dans le cas d'utilisation de forts débits aussi bien de prélèvement que d'alimentation ou dans le cas d'utilisation de solutions à forte viscosité nécessitant l'emploi de canalisations d'accès de diamètre important.

Par le document US-A-4632671, on connaît un dispositif d'aide à l'introduction d'une aiguille à travers un septum implanté comprenant un mandrin de guidage sur lequel coulisse de façon télescopique un embout à introduire, l'embout comportant en arrière de lui une chambre fermée par une membrane perforable que peut traverser le mandrin, ladite membrane étant dirigée sensiblement perpendiculairement à l'axe de l'embout, ladite chambre étant reliée à l'appareil extérieur de traitement, et ledit mandrin d'introduction et guidage de l'embout d'intervention pouvant être retiré en arrière pour dégager le volume intérieur de l'embout après sa mise en place à travers le septum. Cependant, si ce dispositif facilite l'introduction de l'embout à travers le septum et sa connexion avec l'appareil extérieur de traitement, il ne permet en aucune manière l'introduction sans détérioration du septum d'un embout de diamètre plus important qu'usuellement prescrit.

Pour permettre l'introduction d'embouts de gros diamètre, le dispositif d'accès vasculaire implantable conforme à l'invention, du type comportant un septum implanté sous la peau susceptible d'être perforé plusieurs fois au moyen d'aiguilles d'intervention et utilisant un dispositif d'aide à l'introduction du type ci-dessus décrit, se caractérise en ce que plusieurs aiguilles télescopiques coulissant les unes sur les autres et de diamètre progressivement croissant sont prévues pour amener le diamètre de l'ouverture faite dans le septum progressivement jusqu'au diamètre extérieur le plus grand de l'embout par écartement élastique de la paroi de la perforation faite en premier par le mandrin le plus fin.

Selon un mode de réalisation encore plus perfectionné, deux septums sont prévus sur le dispositif implantable, l'un principal fermant la chambre en communication avec l'accès vasculaire lorsqu'une membrane de contrôle est dans une première position de traitement, prélèvement ou alimentation, l'autre secondaire de contrôle fermant une chambre secondaire reliée également à l'accès vasculaire lorsque ladite membrane de contrôle est dans une seconde position avant ou après traitement, l'accès vasculaire étant ainsi en communication soit avec la chambre principale, soit avec la chambre secondaire selon la position occupée par ladite membrane de contrôle.

L'invention et sa mise en oeuvre apparaîtront plus clairement à l'aide de la description qui va suivre faite en référence aux dessins annexés dans lesquels :
la figure 1 montre de façon schématique en coupe transversale le système perfectionné permettant la perforation d'un septum sans détérioration par un embout de diamètre très supérieur au diamètre maximal actuellement utilisable en relation avec ce type de dispositif, lequel embout peut être en relation avec l'appareil extérieur de traitement (dialyse, alimentation, etc...);
la figure 2 montre en vue perspective le dispositif de la figure 1;
la figure 3 montre en coupe tansversale la partie du dispositif implantable comprenant un septum perfectionné utilisable en relation avec le dispositif d'intervention illustré aux figures 1 et 2;
la figure 4 montre à plus grande échelle le détail d'un septum constitué de plusieurs couches superposées comme schématisé à la figure 3;
les figures 5, 6, 7 et 8 sont des vues schématiques en coupe montrant quatre étapes successives d'une intervention pour l'introduction à travers le septum de l'embout d'accès conforme à l'invention permettant la jonction entre l'appareil de traitement et le système vasculaire du patient;
la figure 9 montre en coupe transversale un dispositif à septum implantable perfectionné à deux chambres, le dispositif étant montré dans l'une de ses deux positions de fonctionnement;
la figure 10 montre comme la figure 9 le même dispositif mais dans sa seconde position de fonctionnement;
la figure 11 montre à plus grande échelle en vue éclatée et avec arrachement partiel la superposition de trais pièces essentielles du dispositif illustré à la figure 9.

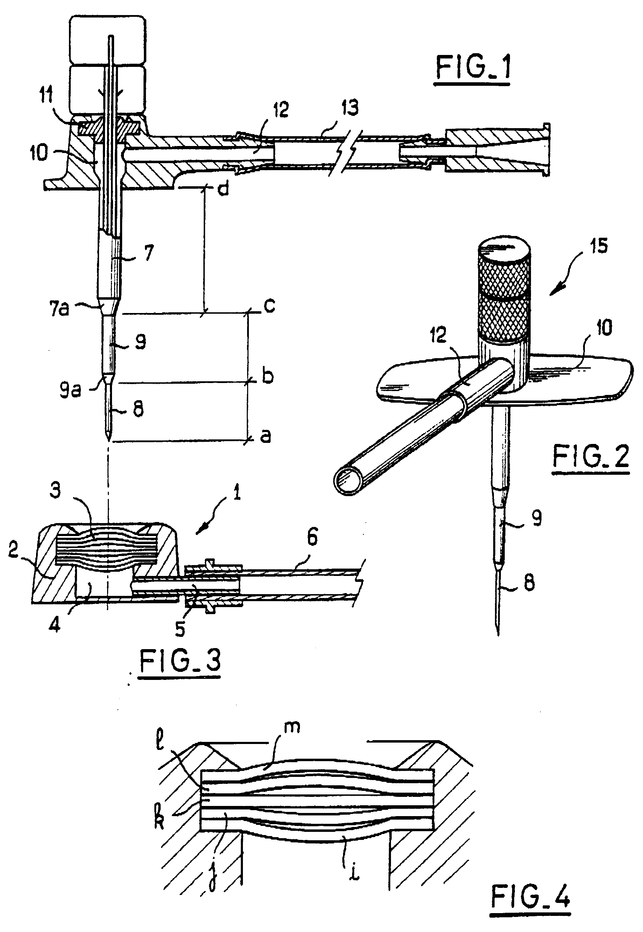

On se reportera tout d'abord aux figures 1, 2 et 3 en regard desquelles on expliquera la mise en oeuvre de l'invention.

A la figure 3 on a illustré un dispositif d'accès vasculaire implantable repéré dans son ensemble 1, comportant essentiellement un corps 2 réalisé en titane ou en résine de synthèse fermé à sa partie supérieure par une cloison élastique souple 3 dite "septum", délimitant ainsi une chambre 4 qui est en relation par un conduit 5 avec un cathéter 6 convenablement relié à un accès vasculaire. En position implantée, l'ensemble du dispositif est logé sous la peau du patient, évitant ainsi les risques infectieux liés aux accès extériorisés.

Comme souligné ci-dessus, l'inconvénient du système est sa limitation au niveau de l'usage, le septum 3 ne pouvant être perforé un grand nombre de fois qu'avec des aiguilles trop fines interdisant l'emploi de ces systèmes pour de nombreuses applications. Dans la pratique, les aiguilles de diamètre maximal pouvant être utilisées ont un diamètre extérieur limité à 0,7 mm.

Conformément à l'invention, on pourra traverser le septum avec un embout tel que repéré 7 présentant un diamètre extérieur très supérieur, par exemple jusqu'à 2 mm, soit un diamètre intérieur de 1,6 mm, du fait que la perforation et la traversée du septum 3 se font de façon progressive successivement au moyen d'un mandrin 8 formé par une aiguille à pointe acérée conique sur laquelle est guidée et coulisse une aiguille 9 creuse de diamètre supérieur à extrémité tronconique 9a qui sert elle-même de guidage à l'embout 7 de diamètre supérieur et d'extrémité tronconique 7a.

On donne ci-après les dimensions d'un dispositif tel qu'illustré à la figure 1 ayant donné toutes satisfactions.

Le mandrin 8 présente un diamètre extérieur de 1,2 mm à sa partie la plus large étant terminé par une fine pointe conique. L'aiguille intermédiaire 9 présente un diamètre extérieur de 1,6 mm, la partie tronconique 9a inclinée à environ 30° sur l'axe permettant le passage progressif du diamètre 1,2 à 1,6 mm. L'embout 7 ayant un diamètre extérieur de 2 mm est terminé par un embout 7a ayant un angle au sommet d'environ 30° permettant de passer progressivement du diamètre extérieur 1,6 mm à 2 mm. Lorsque le dispositif est dans la position (avant intervention) où le mandrin 8 et l'aiguille intermédiaire 9 font saillie sur toute leur longueur à travers le dispositif, le mandrin 8 dépasse d'une longueur ab = 25 mm au delà de l'extrémité de l'aiguille 9, laquelle dépasse elle-même de la longueur bc = 25 mm au delà de l'extrémité de l'embout 7, lequel fait saillie sur la longueur cd de l'ordre de 30 mm en dessous de la plaque d'appui et de saisie en main 10 du dispositif d'intervention.

En ce qui concerne l'embout 7, on voit que celui-ci communique par son extrémité proximale ou arrière avec une chambre 10 fermée en arrière par un septum 11 tel qu'une membrane de silicone, la chambre 10 étant en communication par un tuyau 12 avec un tube 13 de liaison avec l'appareil extérieur de traitement (non représenté).

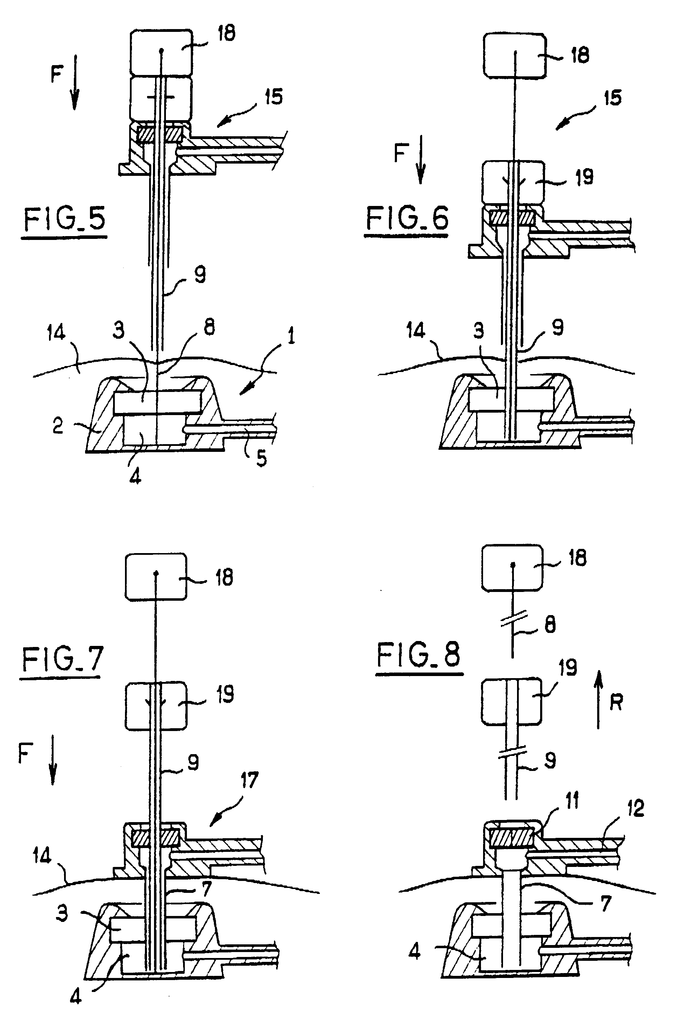

Le fonctionnement du dispositif s'explique aisément notamment en relation avec les schémas des figures 5 à 8.

Dans un premier temps, on introduit le mandrin formant aiguille acérée 8 à travers le septum 3, comme illustré à la figure 5 en poussant l'ensemble du dispositif d'intervention 15 dans l'axe de l'aiguille 8. S'agissant d'un mandrin à fine pointe acérée, il ne forme à travers le septum 3 qu'un trou d'épingle, sans déchirement ou enlèvement de matière. Et par le fait de la progressivité de l'augmentation du diamètre extérieur du mandrin 8, ce trou s'élargit et se déforme élastiquement jusqu'au diamètre extérieur de 1,2 mm du mandrin.

Dans un deuxième temps, comme illustré à la figure 6, on abaisse par rapport à la poignée 18 solidaire du mandrin 8 la poignée 19 solidaire de l'aiguille intermédiaire 9 en faisant coulisser cette dernière sur la mandrin 8 en l'abaissant dans le sens de la flèche F. Ce faisant on continue à déformer élastiquement l'orifice formé dans le septum 3 qui va maintenant s'adapter au diamètre extérieur de 1,6 mm de l'aiguille 9.

Dans un troisième temps on abaisse en le faisant coulisser sur l'aiguille 9 toujours dans le sens de la flèche F l'ensemble 17 lié au mandrin 7, l'orifice formé dans le septum 3 s'élargissant toujours élastiquement jusqu'à 2 mm.

A partir de ce moment, on peut retirer comme illustré à la figure 8 par la flèche R l'aiguille 9 et le mandrin 18 qui avaient été introduits précédemment à travers le septum 11 de l'ensemble 17, ce qui réalise la jonction étanche de l'accès 12 extérieur avec la chambre 4 du dispositif implanté et cela à travers l'embout 7 de diamètre approprié.

Pour améliorer le caractère d'étanchéité du septum 3, on peut avantageusement le constituer comme illustré à la figure 3 et plus précisément à la figure 4 par plusieurs couches superposées de silicone, par exemple douze couches de 0,5 mm (dont cinq seulement ont été représentées et repérées i à m aux dessins pour plus de clarté). On peut avantageusement prévoir entre chaque couche de silicone une intercouche d'un film d'un produit à auto-obturation des perforations tel qu'un silicone qui polymérise au contact de l'humidité. Ainsi, après retrait de l'embout 7, à la suite d'une intervention, toute micro-perforation éventuelle aura tendance à s'auto-obturer.

De même on renforcera avantageusement au moins les couches extrêmes telles que i et m au moyen de fibres synthétiques de façon à limiter les déformations, le tout étant monté en compression dans le corps 2, de façon usuelle.

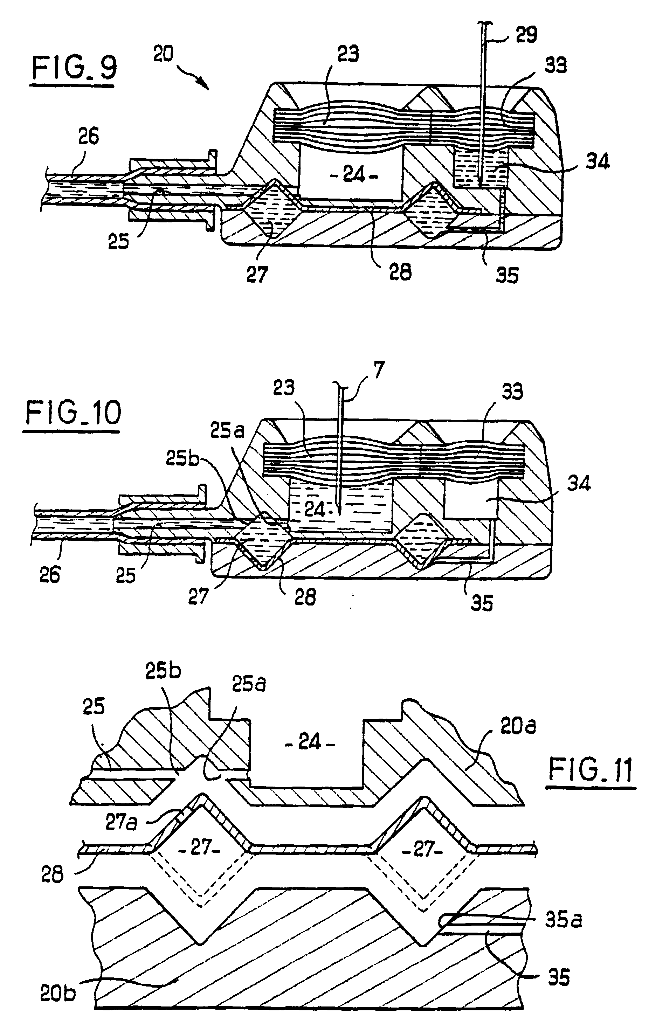

Selon le perfectionnement représenté aux figure 9 à 11, le dispositif implantable, repéré dans son ensemble 20 comporte une chambre principale constituée de façon tout à fait semblable au dispositif 1 précédemment décrit avec un septum 23 fermant une chambre 24 en communication par un tube 25 avec l'accès vasculaire 26.

Mais à côté de ce septum principal 23, on trouve un septum secondaire 33 fermant une chambre 34 pouvant être mise en liaison par une canalisation 35 avec l'accès vasculaire 26 au travers d'une chambre annulaire 27 et du conduit 25, et cela selon la position occupée par une membrane 28 comme il va être décrit.

Comme il apparaît à la figure 11, la membrane 28 qui est avantageusement une membrane souple en silicone est montée comprimée entre la partie supérieure 20a du dispositif et la partie inférieure 20b qui la ferme comme le fond d'un boîtier de montre. Entre le fond 20b et la partie 20a est ménagée une chambre annulaire 27 dans laquelle la membrane 28 joue pouvant prendre deux positions, soit celle dans laquelle elle est tournée vers le haut comme illustré à la figure 9, soit celle dans laquelle elle est tournée vers le bas comme illustré à la figure 10.

Le fonctionnement du dispositif est le suivant.

Si l'on introduit à travers le septum 33 une aiguille d'intervention de type classique fine repérée 29, et que l'on introduit à travers cette aiguille un fluide sous pression, tel que par exemple du sérum physiologique, on remplit la chambre 34 et l'on met sous pression par la canalisation 35 la chambre annulaire 27 en maintenant ou faisant passer la membrane 28 dans la position illustrée à la figure 9. Dans cette position on aperçoit que la membrane 28 obture la canalisation d'accès 25 entre la chambre 24 et l'accès vasculaire 26, au niveau plus précisément du débouché 25a de la chambre 24 vers la chambre annulaire 27. Et dans cette position, la chambre annulaire 27 qui est en communication avec la canalisation 35 est également en communication avec l'accès vasculaire 26 par l'intermédiaire de l'orifice 27a prévu dans la membrane 27 en regard du débouché 25b de la canalisation 25 dans cette chambre annulaire 27.

Si au contraire, comme illustré à la figure 10, on intervient dans la chambre principale 24 au moyen de l'embout 7 d'intervention, on met en pression la chambre annulaire 27 mais cette fois par au-dessus et non par en-dessous et l'on maintient ou fait passer la membrane 28 dans la position illustrée à la figure 10 assurant la communication entre la chambre 24 et l'accès vasculaire 26 et fermant la communication avec la chambre 34 du fait de la membrane 28 qui vient s'appliquer contre le débouché 35a de la canalisation 35 dans la chambre 27.

L'intérêt du dispositif est de permettre notamment en début de traitement l'introduction de faibles quantités d'un fluide tel que du sérum physiologique sous forte pression en vue de permettre le débouchage de l'accès si celui-ci n'ayant pas été utilisé depuis par exemple plusieurs jours se trouvait obturé par des caillots sanguins, et cela sans risque de fuite au niveau du septum principal 23 inactif.

Ce dispositif permet également après toute intervention de replacer la membrane 28 dans la position illustrée à la figure 9 assurant ainsi une double obturation du dispositif avec l'accès vasculaire 26 au niveau de la chambre principale 24.

Etant donné les gros diamètres des embouts tels que 7 pouvant être utilisés, et leur face plane, on prévoira avantageusement que le fond de la chambre 24 est strié ou cannelé de-façon à éviter l'obturation du dispositif notamment lors d'un fonctionnement en aspiration, si l'embout 7 vient porter contre le fond de la chambre 4 ou 24.

L'invention n'est pas limitée aux modes de réalisation illustrés et décrits.

C'est ainsi en particulier que bien que le système d'introduction progressif décrit fasse appel pour retirer le mandrin 8 et l'aiguille 9 intermédiaire de mise en place de l'embout 7 à un système avec septum 11 d'introduction/enlèvement, on pourrait imaginer d'autres systèmes, et par exemple la simple remontée à travers une bague d'étanchéité 11 des aiguilles 8, 9 intermédiaires en position haute dans la chambre 10.

En ce qui concerne la matière constituant le mandrin 8 et les aiguilles 9 et 7, on peut utiliser par exemple des aciers inox ou encore, en particulier pour les aiguilles 9 et 7 une matière plastique compatible telle que FEP (fluoro-éthylène-propylène) ou PTFE (polytétrafluoroéthylène).

## Revendications

1. Dispositif d'accès vasculaire implantables comportant un septum implanté sous la peau susceptible d'être perforé plusieurs fois au moyen d'aiguilles d'intervention par lesquelles sont réalisées les opérations de ponction (prélèvement), dialyse (traitement), traitement chimiothérapique, alimentation en perfusion ou autres, et dans lesquels dispositifs il est prévu de façon à permettre le passage d'aiguilles de plus gros diamètre sans détérioration du septum, pour la traversée du septum un système comportant au moins un mandrin d'introduction et de guidage à pointe fine acérée (8) sur lequel coulisse de façon télescopique une aiguille d'intervention en forme d'embout (7) de diamètre approprié, ledit embout (7) comportant en arrière de lui une chambre (10) fermée sur une face par une membrane perforable (11) que peut traverser ledit mandrin (8) et qui est dirigée sensiblement perpendiculairement à l'axe de l'embout, ladite chambre (10) étant reliée à l'appareil extérieur de traitement, prélèvement ou alimentation, ledit mandrin d'introduction (8) pouvant être, après mise en place de l'embout (7) d'intervention à travers le septum (3), retiré en arrière pour dégager le volume intérieur de l'embout, caractérisés en ce que plusieurs aiguilles télescopiques (8, 7) coulissant les unes sur les autres et de diamètre progressivement croissant sont prévues pour amener le diamètre de l'ouverture faite dans le septum (3) progressivement jusqu'au diamètre extérieur le plus grand de l'embout (7) par écartement élastique de la paroi de la perforation faite en premier par le mandrin le plus fin (8).

2. Dispositif selon la revendication 1, caractérisés en ce que les extrémités de chaque aiguille et embout (7, 9) sont tronconiques de façon à permettre une augmentation progressive du diamètre de l'orifice ouvert dans le septum (3).

3. Dispositif selon l'une des revendications précédentes, caractérisés en ce que le septum (3) est constitué par plusieurs couches superposées de feuilles de silicone rassemblées en compression et en ce qu'entre chaque couche de silicone est prévu un film d'un produit à auto-obturation des perforations tel qu'un silicone qui polymérise au contact de l'humidité.

4. Dispositif selon la revendication 3, caractérisés en ce que certaines des couches, et de préférence au moins les deux couches extrêmes sont renforcées par un tissu de fibres synthétiques.

5. Perfectionnements Dispositif selon l'une quelconque des revendication précédentes, caractérisés en ce que le fond de la chambre du septum comporte des cannelures ou striures.

6. Dispositif selon l'une quelconque des revendications précédentes, caractérisés en ce que deux septums sont prévus sur le dispositif implantable, l'un principal (23) fermant la chambre (24) en communication avec l'accès vasculaire (26) lorsqu'une membrane (28) de contrôle est dans une première position de traitement, prélèvement ou alimentation, l'autre secondaire (33) de contrôle fermant une chambre secondaire (34) reliée également avec l'accès vasculaire (26) lorsque ladite membrane de contrôle (28) est dans une seconde position, avant ou après traitement, l'accès vasculaire étant ainsi en communication soit avec la chambre principale (24), soit avec la chambre secondaire (34) selon la position occupée par ladite membrane de contrôle (28).

7. Dispositif selon la revendication 6, caractérisés en ce que les deux septums (23, 33) sont disposés côte à côte avec leur chambre respective (24, 34) en dessous disposée au dessus d'une chambre annulaire (27) dans laquelle est placée la membrane (28) qui a la forme d'un disque plat au centre et bombé annulairement vers sa périphérie en étant orienté soit vers le haut soit vers le bas, la chambre d'un septum débouchant d'un côté de la membrane dans cette chambre annulaire et l'autre de l'autre côté en obturant la communication avec l'accès vasculaire pour la chambre débouchant du côté supérieur ou inférieur de la chambre annulaire sur lequel s'applique la membrane.

## Claims

1. Implantable vascular access device comprising a septum implanted under the skin and capable of being perforated several times by means of intervention needles which are used to carry out operations involving puncturing (sampling), dialysis (treatment), chemotherapy treatment, perfusion feeding or others, in which device there is provided, for passing through the septum, in such a way as to permit the passage of needles of greater diameter without damaging the septum, a system comprising at least one fine sharp-pointed introducing and guiding mandrel (8) on which there slides in a telescopic manner an intervention needle in the form of a nozzle (7) of appropriate diameter, the said nozzle (7) comprising to its rear a chamber (10) sealed on one face by a perforatable membrane (11) through which the said mandrel (8) can pass and which is directed essentially perpendicular to the axis of the nozzle, the said chamber (10) being connected to the external apparatus for treatment, sampling or feeding, it being possible for the said introducing mandrel (8), after positioning of the intervention nozzle (7) through the septum (3), to be withdrawn rearwards in order to free the internal volume of the nozzle, characterized in that several telescopic needles (8, 7) sliding one upon the other and of progressively increasing diameter are provided in order to bring the diameter of the opening made in the septum (3) progessively to the greatest external diameter of the nozzle (7) by means of elastic spreading of the wall of the perforation made in the first instance by the finest mandrel (8).

2. Device according to Claim 1, characterized in that the ends of each needle and nozzle (7, 9) are truncated so as to allow a progressive increase in the diameter of the orifice opened in the septum (3).

3. Device according to one of the preceding claims, characterized in that the septum (3) consists of several superimposed layers of silicone leaves compressed together, and in that there is provided between each layer of silicone a film of a product for self-closure of the perforations, such as a silicone which polymerises on contact with moisture.

4. Device according to Claim 3, characterized in that some of the layers, and preferably at least the two end layers, are strengthened by means of a tissue of synthetic fibres.

5. Device according to any one of the preceding claims, characterized in that the bottom of the chamber of the septum comprises grooves or ribs.

6. Device according to any one of the preceding claims, characterized in that two septums are provided on the implantable device, the main one (23) sealing the chamber (24) in communication with the vascular access (26) when a control membrane (28) is in a first position for treatment, sampling or feeding, the other secondary control septum (33) sealing a secondary chamber (34) connected likewise to the vascular access (26) when the said control membrane (28) is in a second position, before or after treatment, the vascular access thus being in communication either with the main chamber (24) or with the secondary chamber (34) depending on the position occupied by the said control membrane (28).

7. Device according to Claim 6, characterized in that the two septums (23, 33) are arranged side by side with their respective chambers (24, 34) underneath arranged above an annular chamber (27) in which is placed the membrane (28) which has the form of a flat disc in the centre and is bulged annularly towards its periphery, being oriented either upwards or downwards, the chamber of one septum emptying on one side of the membrane into this annular chamber and the other on the other side sealing the communication with the vascular access for the chamber emptying on the upper or lower side of the annular chamber on which the membrane bears.

## Patentansprüche

1. Implantierbare vaskulare Anschlußvorrichtung mit einem unter die Haut implantierten Septum, das geeignet ist, mehrere Male mittels Eingriffsnadeln durchbohrt zu werden, mit welchen die Punkturtätigkeiten (Entnahme), Dialyse (Behandlung), chemotherapeutische Behandlung, Perfusionsversorgung oder andere durchgeführt werden und in welchen Vorrichtungen, um den Durchgang von Nadeln größeren Durchmessers ohne Beschädigung des Septums zu erlauben, für das Durchqueren des Septums ein System vorgesehen ist, das mindestens einen Dorn mit feiner, scharfer Spitze (8) für die Einleitung und die Führung aufweist, auf welchem teleskopartig eine Eingriffsnadel in Form eines Ansatzstückes (7) von geeignetem Durchmesser gleitet, wobei das Ansatzstück (7) hinter sich eine Kammer (10) aufweist, die auf einer Seite durch eine durchbohrbare Membran (11) geschlossen ist, welche der Dorn (8) durchqueren kann, und die im wesentlichen senkrecht zur Achse des Ansatzstückes gerichtet ist, wobei die Kammer (10) mit dem äußeren Behandlungsapparat verbunden ist, Entnahme oder Versorgung, wobei der Einführdorn (8) nachdem er an dem Eingriffsansatzstück (7) quer durch das Septum (3) angebracht ist, nach hinten zurückgezogen werden kann, um das innere Volumen des Ansatzstückes freizusetzen, dadurch gekennzeichnet, daß mehrere aufeinandergleitende teleskopische Nadeln (8, 7), die einen zunehmend anwachsenden Durchmesser haben, vorgesehen sind, um den Öffnungsdurchmesser, der in dem Septum (3) vorgesehen ist, progressiv bis auf den größten äußeren Durchmesser des Ansatzstückes (7) durch elastischen Abstand von der Wand der Perforation zu bringen, die zuerst durch den feinsten Dorn (8) gemacht ist.

2. Vorrichtung nach Anspruch 1, dadurch gekennzeichnet, daß die Enden jeder Nadel und das Ansatzstück (7, 9) kegelstumpfartig sind, um ein progressives Anwachsen des in dem Septum (3) offenen Öffnungsdurchmessers zu erlauben.

3. Vorrichtung nach einem der vorhergehenden Ansprüche, dadurch gekennzeichnet, daß das Septum (3) aus mehreren übereinandergelagerten Schichten von Siliconblättern besteht, die mit Druck zusammengefügt sind, und daß zwischen jeder Siliconschicht ein Film eines Produktes mit Selbstverschließen der Durchbohrungen, wie z.B. ein Silicon, das bei Kontakt mit Feuchtigkeit polymerisiert, vorgesehen ist.

4. Vorrichtung nach Anspruch 3, dadurch gekennzeichnet, daß bestimmte Schichten und vorzugsweise wenigstens die zwei äußersten Schichten durch ein Gewebe aus synthetischen Fasern verstärkt sind.

5. Vorrichtung nach einem der vorhergehenden Ansprüche, dadurch gekennzeichnet, daß der Boden der Kammer des Septums Riffelungen oder Rillen aufweist.

6. Vorrichtung nach einem der vorhergehenden Ansprüche, dadurch gekennzeichnet, daß zwei Septa auf der implantierbaren Vorrichtung vorgesehen sind, ein hauptsächliches (23), welches die Kammer (24) unter Verbindung mit dem vaskularen Anschluß (26) schließt, während sich eine Steuermembran (28) in einer ersten Behandlungsposition befindet, Entnahme oder Versorgung, das andere sekundäre (33) zur Steuerung, das eine sekundäre Kammer (34) verschließt, die ebenfalls mit dem vaskularen Anschluß (26) verbunden ist, während sich die Steuermembran (28) in einer zweiten Position befindet, vor oder nach Behandlung, wobei der vaskulare Anschluß so entweder mit der Hauptkammer (24) oder mit der Sekundärkammer (34) je nach der von der Steuermembran (28) eingenommenen Stellung in Verbindung steht.

7. Vorrichtung nach Anspruch 6, dadurch gekennzeichnet, daß die zwei Septa (23, 33) Seite an Seite mit ihrer entsprechenden Kammer (24, 34) unten angeordnet sind, die über einer ringförmigen Kammer (27) angeordnet ist, in welcher die Membran (28) angebracht ist, welche im Zentrum die Form einer platten Scheibe hat und zu ihrem Umfang hin ringförmig gewölbt ist, wobei sie entweder nach oben oder nach unten gerichtet ist, wobei die Kammer eines Septums auf der einen Seite der Membran in diese ringförmige Kammer und die andere auf der anderen Seite mündet, wobei die Verbindung mit dem vaskularen Anschluß für die Kammer verschlossen wird, welche auf der oberen oder unteren Seite der ringförmigen Kammer mündet, an welcher die Membran anliegt.
